(19) Europäisches Patentamt
European Patent Office
Office européen des brevets

(11) Veröffentlichungsnummer: **0 225 892 B1**

(12) **EUROPÄISCHE PATENTSCHRIFT**

(45) Veröffentlichungstag der Patentschrift: **16.05.90**

(51) Int. Cl.[5]: **C 12 P 33/00,** C 07 J 1/00

(21) Anmeldenummer: **86903231.8**

(22) Anmeldetag: **02.06.86**

(86) Internationale Anmeldenummer:
**PCT/DE86/00228**

(87) Internationale Veröffentlichungsnummer:
**WO 86/07385 18.12.86 Gazette 86/27**

(54) **VERFAHREN ZUR HERSTELLUNG VON 4-ANDROSTEN-3,17-DION UND 1,4-ANDROSTADIEN-3,17-DION.**

(30) Priorität: **10.06.85 DE 3521111**

(43) Veröffentlichungstag der Anmeldung:
**24.06.87 Patentblatt 87/26**

(45) Bekanntmachung des Hinweises auf die Patenterteilung:
**16.05.90 Patentblatt 90/20**

(84) Benannte Vertragsstaaten:
**AT BE CH DE FR GB IT LI LU NL SE**

(56) Entgegenhaltungen:
**DE-A-2 703 645**
**US-A-3 759 791**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **WEBER, Alfred**
**Schützallee 56**
**D-1000 Berlin 37 (DE)**
Erfinder: **KENNECKE, Mario**
**Taubertstr. 31 f**
**D-1000 Berlin 33 (DE)**

(56) Entgegenhaltungen:

**Experientia, Band 24, Nr. 5, Mai 1968, Birkhäuser Verlag, Basel(CH); G.Ambrus et al.: Über die Hydrierung im Ringsystem ungesättigter Steroide mit Mycobacterium phlei", S. 432**

**Chemical Abstracts, Band 73, Nr. 5, 3 August 1970, Columbus, Ohio (US); M. Nagasawa et al.:"Microbial Transformation of sterols. III.Substrate specificity for cleaving steroid side chains by Arthrobacter simplex", S. 111, Zusammenfassung 22487y**

**Agr. Biol.Chem., Vol. 34, 1970, pp. 798-800**

**EP 0 225 892 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft die in den Patentansprüchen gekennzeichneten Verfahren.

Es ist bekannt, daß zahlreiche Mikroorganismen (so zum Beispiel solche der Gattungen Arthrobacter, Brevibacterium, Microbacterium, Protaminobacter, Bacillus, Norcardia, Streptomyces und insbesondere Mycobacterium) die natürliche Fähigkeit besitzen, natürlich vorkommende 3β-Hydroxy-$\Delta^5$-sterine (wie Cholesterin oder Sitosterin) zu Kohlendioxyd und Wasser abzubauen und daß bei diesem Abbau intermediär 4-Androsten-3,17-dion und 1,4-Androstadien-3,17-dion gebildet werden.

Ferner ist bekannt, daß es mithilfe von Inhibitorzusätzen oder mutierten Mikroorganismen möglich ist, den Abbau der Sterine so zu lenken, daß ein Abbau des gebildeten 4-Androsten-3,17-dions oder 1,4-Androstadien-3,17-dions vermieden wird (siehe deutsche Offenlegungsschriften 15 43 269 und 15 93 327 sowie US-Patentschrift 3,684,657).

Ferner ist bekannt, daß auch Ergosterine mittels Arthrobacter simplex zu 1,4-Androstadien-3,17-dion abgebaut werden kann; im Gegensatz zu dem mikrobiologischen Seitenkettenabbau anderer Sterine erzielt man aber hierbei nur sehr geringe Ausbeuten an Verfahrensprodukt (Agr. Biol. Chem. *34*, 1970, 798—800).

Es wurde nun gefunden, daß man Ergosterin unter den erfindungsgemäßen Bedingungen überraschenderweise unter Erzielung befriedigender Ausbeuten zu 4-Androsten-3,17-dion, oder 1,4-Androstadien-3,17-dion abbauen kann.

Das erfindungsgemäße Verfahren wird unter den gleichen Fermentationsbedingungen durchgeführt, welche man auch bei den bekannten mikrobiologischen Seitenkettenabbau-Reaktionen von Sterinen anwendet.

Erfindungsgemäß wird die Fermentation unter Verwendung von Mikroorganismenkulturen der Gattungen Mycobacterium oder Nocardia durchgeführt.

Als geeignete Mikroorganismen seien beispiels-weise genannt:

Nocardia gardneri IAM—105, Nocardia minima IAM—374, Nocardia corallina IFO—3338,

oder insbesondere die Mikroorganismen Mycobacterium avium IFO—3082, Mycobacterium phlei IFO—3158, Mycobacterium phlei (Institut für Gesundheitswesen, Budapest Nr. 29), Mycobacterium phlei ATCC—354, Mycobacterium smegmatis IFO—3084, Mycobacterium smegmatis ATCC—20, Mycobacterium smegmatis (Institut für Gesundheitswesen, Budapest, Nr. 27), Mycobacterium smegmatis ATCC—19979 und Mycobacterium fortuitum CBS—49566.

Besonders bevorzugt sind als Mikroorganismen Mycobacterium spec. NRRL B—3805, Mycobacterium spec. NRRL B—3683, Mycobacterium phlei NRRL B—8154 und Mycobacterium fortuitum NRRL B—8153 mit deren Hilfe Ergosterin ohne Verwendung zusätzlicher die 9α-Hydroxylierung hemmender Inhibitoren durchgeführt werden kann.

Unter den für diese Mikroorganismen üblicherweise verwendeten Kulturbedingungen werden in einem geeigneten Nährmedium unter Belüften, Submerskulturen angezüchtet. Dann setzt man den Kulturen das Substrat (in einem geeigneten Lösungsmittel gelöst oder vorzugsweise in emulgierter Form) zu und fermentiert, bis eine maximale Substratumwandlung erreicht ist.

Geeignete Substratlösungsmittel sind beispielweise Methanol, Äthanol, Glykolmonomethyläther, Dimethylformamid oder Dimethylsulfoxyd). Die Emulgierung des Substrats kann beispielsweise bewirkt werden, indem man dieses in mikronisierter Form oder in einem mit Wasser mischbaren Lösungsmittel (wie Methanol, Äthanol, Aceton, Glykolmonomethyläther, Dimethylformamid oder Dimethylsulfoxyd) gelöst unter starker Turbulenz in (vorzugsweise entkalktem) Wasser, welches die üblichen Emulgationshilfen enthält eindüst. Geeignete Emulgationshilfen sind nichtionogene Emulgatoren, wie zum Beispiel Äthylenoxyaddukte oder Fettsäureester von Polyglykolen. Als geeignete Emulgatoren seien die handelsüblichen Netzmittel Tegin®, Tween® und Span® beispielsmäßig genannt.

Die optimale Substratkonzentration, Substratzugabezeit und Fermentationsdauer ist von der Struktur des verwendeten Substrates und der Art des verwendeten Mikroorganismus abhängig. Diese Größen müssen, wie dies bei mikrobiologischen Steroidumwandlungen allgemein erforderlich ist, im Einzelfall durch Vorversuche, wie sie dem Fachmann geläufig sind, ermittelt werden.

Die nach dem erfindungsgemäßen Verfahren herstellbaren 4-Androsten-3,17-dion-Derivate der allgemeinen Formel I sind bekanntlich wertvolle Zwischenprodukte die heute gewerbsmäßig zur Synthese phamakologisch wirksamer Steroide verwendet werden.

Die nachfolgenden Beispiele dienen zur Erläuterung des erfindungsgemäßen Verfahrens.

## Beispiel 1

Ein 2 1-Erlenmeyerkolben mit 500 ml sterilem Nährmedium, enthaltend
1% Hefeextrakt
0,45% Dinatriumhydrogenphosphat
0,34% Kaliumdihydrogenphosphat und
0,2% Tween®80
eingestellt auf pH 6,7 —
wird mit einer Suspension einer Mycobacterium spec. NRRL B—3805—Trockenkultur beimpft und 3 Tage lang bei 30°C mit 190 Umdrehungen pro Minute geschüttelt.

20 Erlenmeyerkolben (500 ml) mit jeweils 100 ml sterilem Nährmedium, enthaltend

2,5% Cornsteep liquor
0,3% Diammoniumhydrogenphosphat
0,25% Sojamehl und
0,25% Tween ®80
eingestellt auf pH 7,0 —
werden mit jeweils 5 ml der Mycobacterium spec. — Anzuchtskultur beimpft und 24 Stunden lang bei 30°C mit 220 Umdrehungen pro Minute geschüttelt. Dann setzt man jeder Kultur 30 mg Ergosterin in 1,5 ml Dimethylformamid gelöst zu und fermentiert weitere 96 Stunden lang bei 30°C.

Die vereinigten Kulturen werden mit Äthylenchlorid extrahiert, der Extrakt im Vakuum eingeengt, der Rückstand durch Chromatographie über eine Kieselgelsäule gereinigt und man erhält nach Umkristallisation aus Diisopropyläther 150 mg 4-Androsten-3,17-dion.

Daneben werden 380 mg nicht umgesetztes Ergosterin zurückgewonnen.

Beispiel 2

Unter den Bedingungen des Beispiels 1, jedoch unter Verwendung von Mycobacterium spec. NRRL B—3683 werden 600 mg Ergosterin umgesetzt, aufbereitet und man erhält neben 400 mg unumgesetzten Ergosterin 130 mg 1,4-Androstadien-3,17-dion.

**Patentansprüche**

1. Verfahren zur Herstellung von 4-Androsten-3,17-dion und 1,4-Androstadien-3,17-dion der allgemeinen Formel

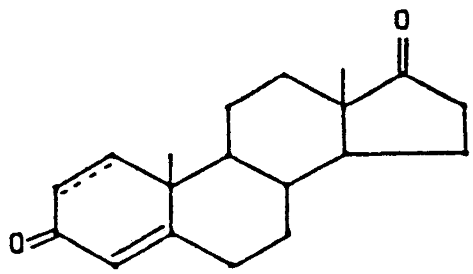

worin ..... eine Einfachbindung oder eine Doppelbindung symbolisiert, dadurch gekennzeichnet, daß man Ergosterin

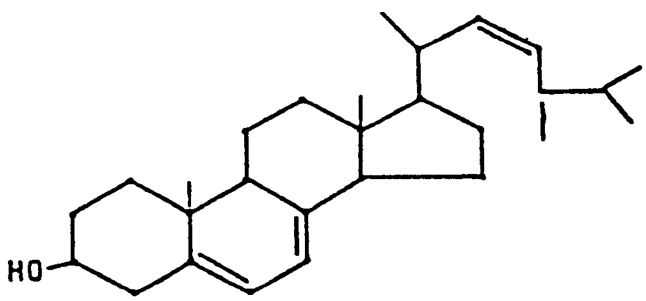

mit einer Kultur eines Mikroorganismus der Gattung Mycobacterium oder Nocardia fermentiert.

2. Verfahren zur Herstellung von 4-Androsten-3,17-dion und 1,4-Androstadien-3,17-dion gemäß Patentanspruch 1, dadurch gekennzeichnet, daß man Ergosterin mit Mycobacterium spec. NRRL B—3805, Mycobacterium spec. NRRL B—3683, Mycobacterium phlei NRRL B—8154 oder Mycobacterium fortuitum NRRL B—8153 fermentiert.

**Revendications**

1. Procédé pour préparer l'androstène-4-dione-3,17 et l'androstadiène-1,4 dione-3,17, composés qui répondent à la formule générale suivante:

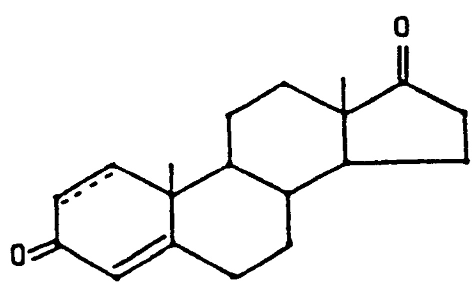

dans laquelle le symbole ..... représente une liaison simple ou une liaison double, procédé caractérisé en ce qu'on fait fermenter l'erqostérol, composé qui répond à la formule:

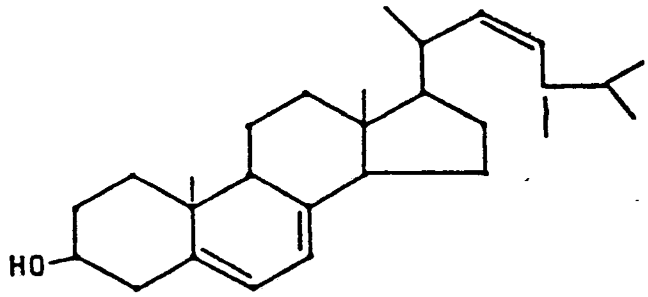

avec une culture d'un micro-organisme du genre Mycobacterium ou Nocardia.

2. Procédé pour préparer l'androstène-4 dione-3,17 et l'androstadiène-1,4 dione-3,17 selon la revendication 1, procédé caractérisé en ce qu'on fait fermenter l'ergostérol avec Mycobacterium spec. NRRL B—3805, Mycobacterium spec. NRRL B—3683, Mycobacterium phlei NRRL B—8154 ou Mycobacterium fortuitum NRRL B—8153.

**Claims**

1. Process for the manufacture of 4-androstene-3,17-dione and 1,4-androstadiene-3,17-dione of the general formula

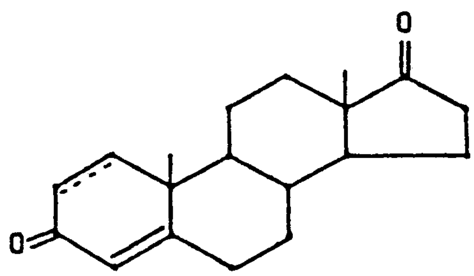

wherein .... represents a single bond or a double bond, characterised in that ergosterin

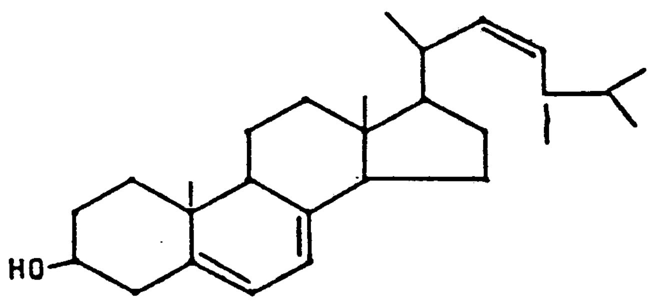

is fermented with a culture of a microorganism of the genus Mycobacterium or Nocardia.

2. Process for the manufacture of 4-androstene-3,17-dione and 1,4-androstadiene-3,17-dione according to patent claim 1, characterised in that ergosterin is fermented with Mycobacterium spec. NRRL B—3805, Mycobacterium spec. NRRL B—3683, Mycobacterium phlei NRRL B—8154 or Mycobacterium fortuitum NRRL B—8153.